Europäisches Patentamt

European Patent Office

Office européen des brevets

(19)

(11) Publication number: **0 089 630**
**A2**

(12) **EUROPEAN PATENT APPLICATION**

(21) Application number: **83102654.7**

(22) Date of filing: **17.03.83**

(51) Int. Cl.³: **G 01 N 27/56**

(30) Priority: **19.03.82 JP 45154/82**
**19.03.82 JP 45155/82**

(43) Date of publication of application:
**28.09.83 Bulletin 83/39**

(84) Designated Contracting States:
**CH DE FR GB IT LI NL SE**

(71) Applicant: **Hitachi, Ltd.**
**5-1, Marunouchi 1-chome**
**Chiyoda-ku Tokyo 100(JP)**

(72) Inventor: **Oyama, Yoshishige**
**3-24-18, Higashioshima**
**Katsuta-shi Ibaraki-ken(JP)**

(72) Inventor: **Kirisawa, Tadashi**
**3600-429, Nakane**
**Katsuta-shi Ibaraki-ken(JP)**

(72) Inventor: **Ohsuga, Minoru**
**Yuho-ryo, 6-20-3 Ayukawa-cho**
**Hitachi-shi Ibaraki-ken(JP)**

(74) Representative: **Patentanwälte Beetz sen. - Beetz jun.**
**Timpe - Siegfried - Schmitt-Fumian**
**Steinsdorfstrasse 10**
**D-8000 München 22(DE)**

(54) Device for measuring oxygen concentration in exhaust gas.

(57) An oxygen concentration measuring device for measuring oxygen concentration in exhaust gas from a combustor, wherein the oxygen concentration in the exhaust gas is made through comparison with a reference value which is obtained through detection of oxygen concentration in the atmosphere, either by creating the same condition as the atmosphere in the exhaust pipe through temporarily interrupting the fuel supply to the combustor or by temporarily introducing the atmosphere directly into the exhaust pipe. The use of the oxygen concentration in the atmosphere as the reference value permits an accurate measurement of oxygen concentration in the exhaust gas for a long period of time regardless of extent of contamination of the detector element by contaminants suspended by the exhaust gas.

*FIG. 1*

Title of the Invention

DEVICE FOR MEASURING OXYGEN CONCENTRATION IN
EXHAUST GAS

Background of the Invention

The present invention relates to a device for measuring oxygen concentration in exhaust gas coming from a combustor and, more particularly, to an oxygen concentration measuring device employing an oxygen concentration detector of the type in which electric current is supplied to a solid electrolyte having an oxygen-ion conductivity and the oxygen concentration is detected through measurement of the rate of passage of oxygen through diffusion resistor.

For attaining higher combustion efficiency in combustors such as diesel engines, gasoline engines and so forth, it is necessary to precisely control the air-fuel ratio of the mixture, which in turn requires an accurate measurement of oxygen concentration in the exhaust gas and a feed back of the result of the measurement to the fuel supply system.

Hitherto, oxygen concentration detectors of oxygen pump type as shown, for example, in SAE paper 810433 or U.S.P.4,158,166 have been used for the purpose mentioned above. In the oxygen concentration detector of the oxygen pump type, electric current is supplied to an oxygen-ion conductive solid electrolyte to transfer oxygen, and the oxygen concentration is detected as the rate of transfer of oxygen caused by the difference in concentration across orifices or porous member.

Exhaust gases from combustors such as diesel engines or gasoline engines inevitably contain solid components such as particles of lead, carbon and so forth, as well as mist of heavy oil, besides the pure gaseous components. These solid matters inconveniently attach to the orifices of porous body to cause a change in the diffusion resistance. The change in the diffusion resistance causes a change in the output signal, i.e. measurement error, to degrade the accuracy of measurement. Usually, diesel engines and gasoline engines are used for long period of time and, therefore, the measurement error is incurred during the long use for the reason explained above and, accordingly, there has been a demand for development of suitable measure for overcoming this problem. U.S.P. 3,907,657 discloses, as one of such measures, an analyzing apparatus and method which are not influenced substantially by slight change in the diffusion resistance. These apparatus and method, however, cannot apply practically to the measurement of oxygen concentration in exhaust gas from combustor, beause such measurement requires a high precision and a large measuring electric current.

Summary of the Invention

Accordigly, an object of the invention is to provide a device for measuring oxygen concentration in exhaust gas from a combustor, capable of measuring the oxygen concentration without any substantial measurement error even when the diffusion resistance is varied due to a secular change.

Another object of the invention is to provide a device for measuring oxygen concentration in exhaust gas from a combustor in which the measurement accuracy is not influenced by deposition of particles such as of lead, carbon, heavy oil mist and so forth on the diffusion resistor of the detector.

To these ends, according to the invention, there is provided an oxygen concentration measuring device having means for temporarily interrupting the fuel supply to the combustor to form an exhaust gas composition equal to that of the natural atmosphere or for directly introducing the atmosphere into the portion where he measurement is made, means for storing, as a reference signal, the oxygen concentration measured during operation of the above-mentioned means, and means for determining the oxygen concentration in the exhaust gas through comparison with the reference signal.

Brief Description of the Drawings

Fig. 1 is a sectional view of an embodiment of oxygen concentration measuring apparatus in accordance with the present invention;

Fig. 2 is a sectional view of another embodiment of the oxygen concentration measuring apparatus of the invention; and

Fig. 3 is a chart showing, by way of example, how the detection electric current and the voltage are changed in relation to the oxygen concentration.

Description of the Preferred Embodiments

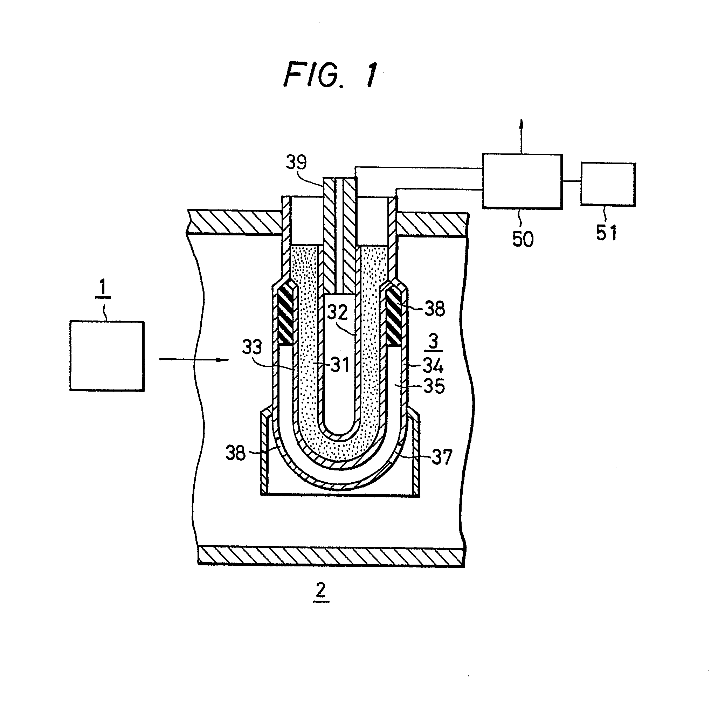

Referring first to Fig. 1, a combustor 1 which is, for example, a gasoline engine or a diesel engine has an exhaust pipe 2 through which the exhaust gas is discharged. An oxygen concentration detector 3 is disposed at a predetermined portion of the exhaust pipe 2. The oxygen concentration detector 3 is of so-called oxygen pump type employing a solid electrolyte 31 which is, for example, a mixture of $ZrO_2$-$Y_2O_3$, although any other material having oxygen-ion conductivity can be used as the solid electrolyte 31. Porous platinum electrodes 32 and 33 are provided on both sides of the electrolyte 31. The electrolyte 31 provided with the platinum electrodes is accomodated by a cover 34 made of a heat-resistant material, so that the electrode 33 of the electrolyte may not be directly subjected to the exhaust gas. Orifices 36 and 37 serveable as diffusion resistors are disposed at suitable portions of the cover 34. The exhaust gas as the measuring object is introduced through the orifices 36 and 37 into a chamber 35 which is defined between the cover 34 and the side of the electrolyte 31 carrying the electrode 33. In the embodiment shown in Fig. 1, the orifices 36 and 37 have a diameter of about 0.5 mm and a length of about 2 mm. The orifices 36 and 37, which are intended for imparting diffusion resistance, may be substituted by porous material such as ceramics.

The detector 3 is provided at a portion thereof with a heater 38 for heating the electrolyte 31 and the cover 34 up to 600 to 1000°C. The heater 38 has an insulating ceramics such as silicon nitride in which a tungsten wire is embedded.

The detector 3 is mounted on a predetermined portion as the cover 34 is fixed to the exhaust pipe 2 by means of, for example, screws. The electrode 32 is connected to a hollow terminal 39 the interior of which communicates with the atmosphere, while the electrode 33 is connected to the cover 34. A strainer 40 is provided at the outer side of the orifices 36 and 37 for preventing attaching of contaminants suspended by the exhaust gas to the orifices 36 and 37.

The terminal 39 and the cover 34 are electrically connected to a detection electric circuit 50 for detecting the measuring electric current. The detection electric circuit 50 is connected to a rewritable memory device (microprocessor) 51, and performs a computation for determining the oxygen concentration as will be explained later.

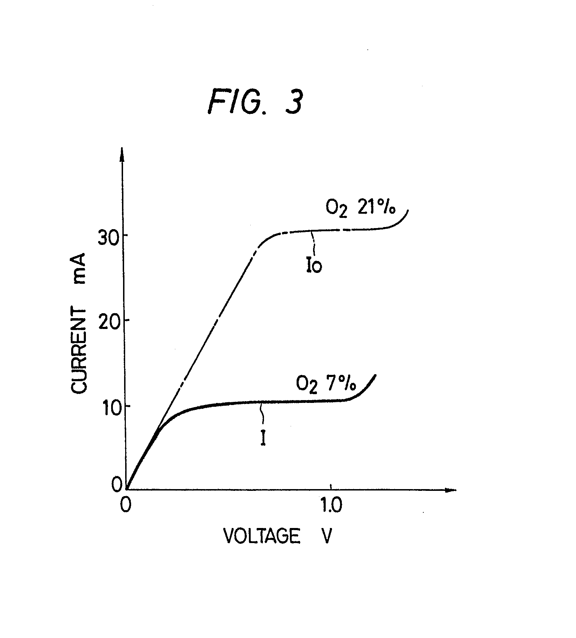

Fig. 3 shows the result of measurement of the voltage and electric current across the electrodes 32 and 33 in the detector 3, for each of the atmosphere containing 21% oxygen and exhaust gas containing 7% oxygen. It will be seen that the electric current is changed in proportion to the oxygen concentration of the measuring object.

Using the oxygen concentration measuring device of the invention described hereinbefore, the measurement of oxygen concentration in exhaust gas is conducted in the following manner.

First of all, electric current is supplied to the heater 38 so as to heat the electrolyte 31 and the cover 34 to a temperature of 600 to $1000^{o}C$. As this high temperature is reached, the oxygen-ion conductivity of the electrolyte 31 is increased to enable the detector to work. Then, positive and negative voltages of about 2V are applied to the electrodes 32 and the electrode 33, respectively, so that electric current is produced to generate an oxygen pump effect to transfer the oxygen in the chamber towards the electrode 32. The oxygen thus transferred is discharged to the atmosphere through the hollow of the terminal 39. As a result of the oxygen pumping effect, the partial pressure of oxygen in the chamber 35 is lowered to permit new oxygen to be diffused into the chamber 35 through the orifices 36 and 37.

Representing the oxygen concentration (partial pressure) at the outside of the orifices 36 and 37 by $P_A$ and the oxygen concentration (partial pressure) inside the chamber 35 by $P_V$, the total flow rate of oxygen flowing into the chamber 35 through the orifices 36 and 37 is proportional to $K(P_A - P_V)$, where K represents the diffusion resistance which varies depending on the shape of the orifices 36 and 37 as well as by temperature and pressure.   According to Faraday's law, the flow rate of oxygen is proportional to the current I flowing in the solid electrolyte 31.

$$I \propto K (P_A - P_V) \qquad \ldots\ldots (1)$$

As the electric current in this state is increased by an increase of the voltage applied, the oxygen concentration (partial pressure) in the chamber 35 is decreased.   The following relationship is derived from the Gibs' law of free energy.

$$P_V = P_A e^{- \frac{4EF}{RT}} \qquad \ldots\ldots (2)$$

where, E represents voltage, F represents Faraday's constant, R represents gas constant and T represents temperature.   From formula (2) above, it is understood that the voltage E is decreased as the oxygen concentration (partial pressure) $P_V$ is

lowered. Namely, the current I is increased as the oxygen concentration $P_V$ is decreased as a result of increase in the voltage E. The value of the electric current I, however, cannot exceed the amount proportional to $KP_A$ as will be understood from formula (1). Namely, .in the region around $P_V \doteq 0$, the electric current I is proportional to $KP_A$ and, therefore, the oxygen concentration $P_A$ is known from the value of the current I. As will be understood from formula (1), the electric current I is changed as the diffusion resistance K is changed. As stated before, in the detector disposed in the exhaust system of a combustor, the detector is inevitably contaminated by non-gaseous components in the exhaust gas, and the electric current I is varied as a result of contamination to incur a measurement error.

This problem, however, is obviated in the oxygen concentration measuring device of the invention, as will be fully understood from the following description. Namely, the oxygen concentration measuring device of the invention has a rewritable memory device 51 besides the detection electric circuit 50. In measuring the oxygen concentration, the fuel supply to the combustor 1 is temporarily interrupted for a while until the condition same as the atmosphere, i.e. a gaseous condition having oxygen partial pressure of 21%, is established in the exhaust pipe 2, and the value $I_0$ of the electric current measured in this state is stored in the rewritable memory device 51. When the oxygen concentration measuring device of the invention is applied to an automotive gasoline or diesel engine, the interruption of the fuel supply is made preferably during the deceleration of the automobile so as not to disturb the driving condition of the automobile. The interruption of the fuel supply can be made by known means although the drawings lack illustration of means for interrupting the fuel supply.

Then, the supply of fuel is started again and the electric current I is measured in the operating condition of the cmbustor. The oxygen concentration in this state is determined by comparing this electric current I with the electric current $I_0$ measured during interruption of fuel supply and now stored in the rewritable memory device 51 and making a computation in accordance with the following formula (3).

$$X = 21 \times \frac{I}{I_0} \quad (\%) \qquad \qquad \ldots \ldots (3)$$

It will be understood that, since the currents $I_0$ and I are measured under the same diffusion resistance K, the oxygen concentration X is completely freed from the extent of contamination of the orifices 37 and 38.

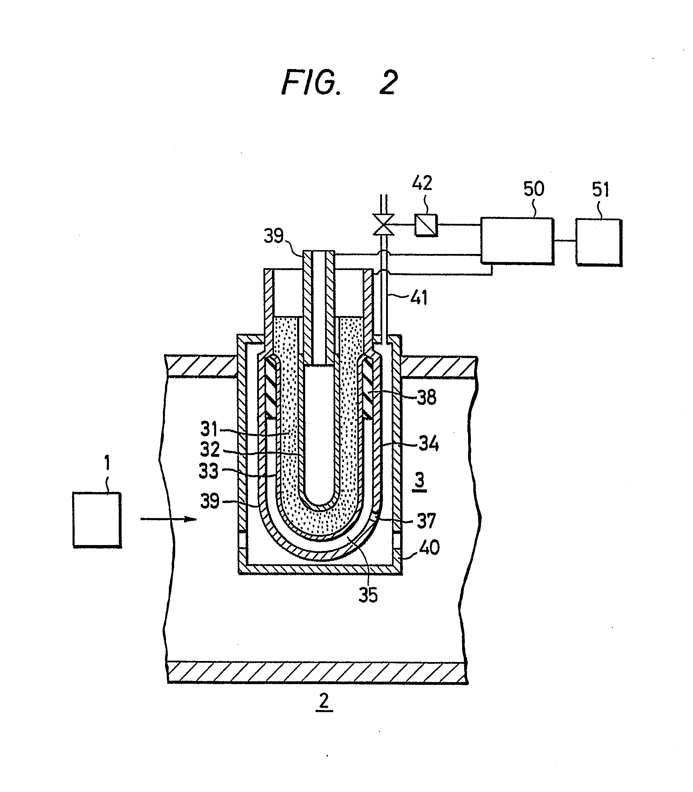

Fig. 2 shows another embodiment of the invention, in which the whole part of the detector is covered by a strainer 40, and a chamber 39 defined by the strainer 40 and the cover 34 is communicated with the atmosphere through a pipe 41 and a solenoid valve 42. Thus, in this embodiment, the solenoid valve 42 is opened to permit direct ingtroduction of atmosphere into the chamber 39, insteadly of temporarily interrupting the fuel supply to the combustor. By opening the solenoid valve 42 at the time of starting and stopping of the combustor 1, it is possible to obviate the contamination by the non-gaseous components which is heavy particularly when the combustor is started and stopped.

In this embodiment of the invention, it is possible to directly measure the concentration of oxygen in the atmosphere introduced into the combustor, and the electric current $I_0$ measured in this state is stored in the rewritable memory device 51. Then, the oxygen concentration in the exhaust gas is measured after closing the solenoid valve, and the exhaust gas concentration X is determined through a computation in accordance with the formula (3).

As has been stated, in the oxygen concentration measuring device in accordance with the invention, the electric current I obtained through the measurement is always compared with and corrected by the electric current value $I_0$ obtained through the measurement of the oxygen concentration in the atmosphere so that a high precision of measurement is maintained over a long period of time, regardless of attaching of contaminants to the diffusion resistor.

Although the invention has been described through specific terms, it is to be noted here that the described embodiments are not exclusive and various changes and modifications may be imparted thereto without departing from the scope of the invention which is limited solely by the appended claims.

What is Claimed is:

1. An oxygen concentration measuring device for measuring oxygen concentration in exhaust gas, having an oxygen concentration detector of the type including a closed chamber (35) having a side wall (34) a portion of which is constituted by an oxygen-conductive solid electrolyte (31) while another part is constituted by a diffusion resistor (36, 37), so that the oxygen in said closed chamber is transfered to the outside as electric current is supplied to said solid electrolyte (31) to produce a difference of oxygen concentration between the inside and the outside of said closed chamber (35) so that the oxygen concentration is detected through measurement of the flow rate of oxygen permeating through said diffusion resistor (36, 37) and the value of electric current, said oxygen concentration measuring device comprising: means (39) for temporarily introducing ambient atmosphere to said oxygen concentration detector, and means (51) for storing the value of the oxygen concentration in the introduced atmosphere, whereby the oxygen concentration in the gas to be measured is determined through comparison of the measured oxygen concentration with the oxygen concentration of the atmosphere stored in said memory device (51).

2. An oxygen concentration measuring device according to claim 1, wherein said means for temporarily introducing ambient atmosphere
includes a fuel cut-off device for temporarily interrupting
the fuel supply to said combustor.


3. An oxygen concentration measuring device according to claim 1,
wherein said means for temporarily introducing ambient
atmosphere includes a cover (40) surrounding the whole part
of said oxygen concentration detector, a chamber (39) defined
between said cover and said oxygen concentration detector,
an air inlet (41) through which said chamber is communicated
with the atmosphere and a stop valve provided in said air
inlet and adapted to control the introduction of said
ambient atmosphere.

FIG. 1

FIG. 2

# FIG. 3